# EUROPÄISCHE PATENTSCHRIFT

(11) **EP 1 061 851 B1**
(45) Veröffentlichungstag und Bekanntmachung des Hinweises auf die Patenterteilung: **20.02.2002**
(21) Anmeldenummer: 98922614.7
(22) Anmeldetag: 07.03.1998
(51) Int. Cl.: A61B 5/11

(54) **VERFAHREN UND VORRICHTUNG ZUR AUSWERTUNG EINES BEWEGUNGSMUSTERS**
METHOD AND DEVICE FOR EVALUATING AN KINETIC PATTERN
PROCEDE ET DISPOSITIF POUR L'EVALUATION D'UN SCHEMA CINEMATIQUE

(43) Veröffentlichungstag der Anmeldung: 27.12.2000
(73) Patentinhaber: Claussen, Claus-Frenz, 97688 Bad Kissingen (DE)
(72) Erfinder: CLAUSSEN, Claus-Frenz, D-97688 Bad Kissingen (DE); HARALANOV, Svetlozar, 1606 Sofia (BG)
(74) Vertreter: Tergau & Pohl Patentanwälte
(86) Internationale Anmeldenummer: EP9801341
(87) Internationale Veröffentlichungsnummer: WO9944502

(56) Entgegenhaltungen:
- WO-A-91/15148
- DE-A- 3 829 885
- US-A- 4 631 676
- SAFAEE-RAD R ET AL: "THREE-DIMENSIONAL MEASUREMENT SYSTEM FOR FUNCTIONAL ARM MOTION STUDY" MEDICAL AND BIOLOGICAL ENGINEERING AND COMPUTING, Bd. 28, Nr. 6, 1. November 1990, Seiten 569-573, XP000220093
- CARRERA D J ET AL: "THE RELIABILITY OF POSTURAL SWAY MEASURES USING THE 2SPACE TRACKER" CLINICAL BIOMECHANICS, Bd. 11, Nr. 6, 1. September 1996, Seiten 361-363, XP000599794
- "Forschungsbericht Cranio-Corpo-Graphie (CCG)" aus der Schriftreihe des Hauptverbandes der gewerblichen Berufsgenossenschaften e.V.; Juni 1986: Seiten 7 bis 61; ISBN 3-88383-126-3

## Beschreibung

Die Erfindung bezieht sich auf ein Verfahren zur Auswertung eines Bewegungsmusters, das anhand einer Anzahl von mit dem Körper eines Probanden mitbewegten Markierern aufgenommen wird. Sie bezieht sich weiter auf eine entsprechende Vorrichtung zur Durchführung des Verfahrens. Unter Bewegungsmuster wird hierbei das Muster der Kopf- und Rumpfbewegung (Cranio-Corpo-Gramm) verstanden.

Gleichgewichtsfunktionsstörungen treten als Haupt- oder Folgeerscheinung einer Vielzahl von pathologischen Befunden auf. Hierbei handelt es sich einerseits um anlage-bedingte Phänomene, wie z. B. den Höhenschwindel, oder um bei einem Unfall hervorgerufene Schädigungen, z. B. einem Schleudertrauma. Andererseits sind zahlreiche Krankheitsbilder mit reversiblen oder irreversiblen Störungen der Gleichgewichtssteuerung verbunden, darunter auch psychosomatische Erkrankungen, wie beispielsweise Schizophrenie, Demenzerscheinungen, Depression und das Parkinson-Syndrom.

Die Einhaltung des Gleichgewichts erfordert insbesondere beim stehenden Menschen einen hochkomplizierten Regelmechanismus, an dem zusätzlich zu dem im Innenohr gelegenen Gleichgewichtsorgan (Vestibufarapparat) vor allem die Augen und die Ohren sowie Tastrezeptoren und verschiedene Bereiche des Gehirns beteiligt sind. In die zu Diagnosezwecken herangezogene Untersuchungsmethode der sogenannten "Cranio-Corpo-Graphie" fließt die Erkenntnis ein, daß Störungen in verschiedenen Bereichen der an der Gleichgewichtssteuerung beteiligten Organe zu einem jeweils charakteristischen Bewegungsmuster eines zu untersuchenden Probanden führen. Durch Beobachtung des Bewegungsmusters läßt sich gemäß der Cranio-Corpo-Graphie empirisch die Störung innerhalb der Gleichgewichtssteuerung lokalisieren. Daraus wiederum können Rückschlüsse auf eine die Gleichgewichtsfunktionsstörung auslösende Krankheit gezogen werden.

Gemäß der aus der Schriftreihe des Hauptverbandes der gewerblichen Berufsgenossenschaften e. V. erschienenen Druckschrift "Forschungsbericht Cranio-Corpo-Graphie (CCG)", ISBN 3-88383-126-3 (Juni 1986)", bekannten Einrichtung wird die Kopf- und Rumpfbewegung des Probanden mittels Markierern in Form von Glühlampen sichtbar gemacht, von welchen je einer an beiden Schultern des Probanden und über dessen Stirn sowie dessen Hinterhaupt angebracht ist. Die Bewegung jedes Markierers in der horizontalen Ebene wird von einer über dem Probanden angeordneten Kamera in Dauerbelichtung als Leuchtspur in einem sogenannten Cranio-Corpo-Gramm photographisch festgehalten. Die Leuchtspuren werden nach Durchführung des Experimentes auf der Photographie manuell ausgewertet.

Die manuelle Auswertung der Aufnahmen, die entweder durch Vermessung der Geometrie der Leuchtspuren oder durch assotiative Verbindung des komplexen Bewegungsmusters mit als "Grapho-Element" bezeichneten Vergleichsmustern erfolgt, ist jedoch mit einem erheblichen Zeitaufwand verbunden. Ein wesentlicher Nachteil besteht zudem darin, daß bei der photographischen Aufnahme der Markiererbewegung ein Teil der im Experiment erzeugten Information verloren ist. So sind auf der Photographie lediglich die horizontalen Komponenten der Markiererbewegung zu erkennen. Es kann somit keine Aussage über vertikale Bewegungen und die absolute Höhe eines Markierers im Raum getroffen werden. Deshalb ist eine aufwendige Eichung der Photographie erforderlich, um Cranio-Corpo-Gramme unterschiedlich großer Probanden überhaupt vergleichen zu können. Da die Leuchtspuren aller Markierer auf einer einzelnen Photographie enthalten sind, kommt es häufig zu Abdeckungen durch Überschneidungen der Leuchtspuren, die die Charakterisierung einer einzelnen Leuchtspur erschweren oder sogar unmöglich machen. Information geht auch in dem direkt unterhalb der Kamera gelegenen toten Winkel verloren, in welchem die Kamera in den zwischen dem Spiegel und einem Markierer verlaufenden Strahlengang hineinsteht.

Aus der DE 38 29 885 C2 ist eine Einrichtung bekannt, in welcher anstelle einer Kamera eine über dem Probanden angebrachte Anordnung von Photozellen zur Aufnahme der Leuchtspuren eingesetzt wird. Durch diese Anordnung entfällt der tote Winkel. Die Leuchtspuren werden dort mittels eines Digital-Computers hinsichtlich einer Berechnung der für die Cranio-Corpo-Graphie relevanten Bewegungsabweichungen analysiert. Eine Auswertung im Hinblick auf eine Interpretation erfaßter Bewegungsmuster ist dort jedoch nicht vorgesehen.

Der Erfindung liegt daher die Aufgabe zugrunde, ein Verfahren zur Auswertung von anhand einer Anzahl von mit dem Körper eines Probanden mitbewegten Markierern aufgenommenen Bewegungungsmustern anzugeben, bei dem das Bewegungsmuster unter Erhaltung einer besonders hohen Informationsdichte mit geringem Zeitaufwand ausgewertet wird. Desweiteren soll eine besonders geeignete Vorrichtung zur automatischen Durchführung des Verfahrens angegeben werden.

Bezüglich des Verfahrens wird diese Aufgabe erfindungsgemäß gelöst durch die Merkmale des Anspruchs 1. Dabei wird die Ortskurve jedes Markierers im dreidimensionalen Raum zeitaufgelöst erfaßt und als Datenfeld eines allen Markierern gemeinsamen Datensatzes gespeichert. Die das Bewegungsmuster repräsentierenden Ortskurven werden danach mittels einer Datenverarbeitungsanlage anhand von aus dem Datensatz abgeleiteten Kenngrößen charakterisiert. Anschließend wird jede Kenngröße mit aus einem hinterlegten Referenzdatensatz entsprechend abgeleiteten Referenzgrößen nach Art einer Mustererkennung verglichen. Die oder jede Referenzgröße wird dazu bei einer Referenzmessung als Kenngröße eines Referenz-Bewegungsmusters ermittelt. Unter Kenngröße bzw. Referenzgröße wird hierbei jede aus den Ortskurven ableitbare Information zur Charakterisierung des Bewegungsmusters im Hinblick auf eine Mustererkennung verstanden.

Die Erfindung geht dabei von der Überlegung aus, daß aus einem Cranio-Corpo-Gramm ableitbare quasi prädiagnostische Aussagen auf empirisch gewonnenen Erfahrungswerten beruhen. Für eine zuverlässige Diagnose eines Krankheitsbildes aufgrund eines beobachteten Bewegungsmusters bedarf es daher eine Vielzahl von Referenzuntersuchungen, da im Bewegungsmuster ebenfalls enthaltene individuelle Eigenschaften von pathologischen Eigenschaften getrennt werden müssen. Die für eine statistische Absicherung der Erkenntnisse zu verarbeitende Datenmenge erreicht eine derartige Komplexität, daß zuverlässige Aussagen nur mit hohem Zeitaufwand möglich sind. Eine zeitaufwendige Auswertung einer Untersuchung macht jedoch eine Anwendung im klinischen Bereich unrentabel. Darüber hinaus ist es wünschenswert, den Informationsverlust bei der Aufzeichnung eines Experiments so gering wie möglich zu halten, zumal häufig neue Erkenntnisse eine erneute Auswertung alter Untersuchungen oder Experimente unter einem neuen Gesichtspunkt erforderlich machen. Durch eine automatische Mustererkennung mittels eines Rechners oder einer Datenverarbeitungsanlage kann eine besonders detaillierte Analyse eines Bewegungsmusters mit im Ergebnis einer Typisierung von Bewegungsgestalten erreicht werden. Dieses Ergenis kann erkanntermaßen bereits eine Wahrscheinlichkeitsaussage für eine spätere Diagnose beinhalten.

Infolge der dreidimensionalen, zeitaufgelösten Aufzeichnung enthält der Datensatz die gesamte Information des Experiments. Insbesondere können die Ortskurven der Markierer stets getrennt voneinander ausgewertet werden. Außerdem ist der Zeitverlauf des Bewegungsmusters besonders einfach zu analysieren. Aufgrund der Leistungsfähigkeit moderner Datenverarbeitungssysteme ist der Zeitaufwand gegenüber einer manuellen Auswertung des Bewegungsmusters erheblich reduziert.

Die Kenngrößen werden aus einer Projektion mindestens einer Ortskurve auf eine der Grundebenen eines karthesischen Koordinatensystems abgeleitet. Gegenüber einem aus der WO 91/15148 bekannten Verfahren zur Bewegungsbestimmung der Halswirbelsäule anhand von aus dreidimensionalen Bildinformationen einer Kopfbewegung ermittelten räumlichen Darstellungen der Kopfrotationsachse läßt sich die Ortskurve in einer zweidimensionalen Projektion besonders übersichtlich, z. B. auf einem Bildschirm, darstellen und effizient ausworten. Dies ist insbesondere deshalb besonders vorteilhaft, da das für diagnostische oder prädiagnostische Zwecke herangezogene Auswertungsergebnis bezüglich jedes Verfahrensschritts nachvollziehbar sein soll.

Typischerweise zeigt das in einem Cranio-Corpo-Gramm beobachtete Bewegungsmuster eine periodische Struktur, die von einer Körperschwankung des Probanden hervorgerufen wird. Bei einem sogenannten Schrit-Test (nach Unterberger/Fukuda), bei dem der Proband Schrittbewegungen ausführt, entspricht ein halber Schwankungszyklus einem Schritt des Probanden. Zur Ableitung von Kenngrößen, die das Bewegungsmuster besonders detailliert beschreiben, ist es zweckmäßig, die Ortskurve entsprechend ihrer Periodizität in Sequenzen zu unterteilen. Daher werden bestimmte Kenngrößen aus einer einzelnen Sequenz ermittelt. Derartige Kenngrößen sind beispielsweise die Amplitude, die Schwankungsdauer und die quer zur Schwankungsrichtung zurückgelegte Strecke (Schrittweite).

Da die einer Körperschwankung innewohnende Periodizität erkanntermaßen keine exakte Periodizität im mathematischen Sinne ist, sind die einzelnen Sequenzen nicht identisch, sondern nur ähnlich. Zweckmäßigerweise wird daher zusätzlich oder alternativ zu einer aus einer einzelnen Sequenz ermittelten Kenngröße ("single-step"-Analyse) eine entsprechende Kenngröße aus einer Anzahl von Sequenzen ermittelt und statistisch in Form eines Mittelwerts mit dessen Standardabweichung angegeben. Auf diese Weise lassen sich Aussagen über die Regelmäßigkeit einer Körperschwankung ableiten.

Quantitative Angaben hinsichtlich der Regelmäßigkeit einer Körperschwankung werden zweckmäßigerweise durch Ermittlung der Amplitudenverteilung und/oder der Frequenzverteilung mindestens einer Ortskurve erhalten. So wird insbesondere die Frequenzverteilung mittels eines Spektralanalyseverfahrens, z. B. mit der sog. "Schnellen Fourrier Transformation", als Kenngröße aus dem Datensatz abgeleitet.

Als weitere zweckmäßige Kenngrößen wird die Schwerpunktsbewegung des Körpers ermittelt. Dazu wird aus dem oder jedem Datenfeld des Datensatzes der Abstand zwischen der Anfangsposition und der Endposition des jeweiligen Markierers berechnet. Daraus kann z. B. der Körperabweichungswinkel bestimmt werden. Zusätzlich werden anhand der relativen Positionen der Markierer zueinander ermittelte Kenngrößen herangezogen, die die Ausrichtung der Körperteile im Raum und deren Stellung, insbesondere des Kopfes und der Schultern, relativ zueinander repräsentieren. Diese Kenngrößen sind insbesondere der Körpereigenspin und der Torticolliswinkel, d. h. die Verstellung des Kopfes gegenüber den Schultern. Ferner kann die Länge der oder jeder Ortskurve und damit die vom jeweiligen Markierer zurückgelegte Gesamtstrecke als Kenngröße ermittelt werden.

Anhand eines Mustervergleichs von aus aktuellen Datensätzen und aus gespeicherten Referenzdatensätzen abgeleiteten Mustern in Form von Graphoelementen können, insbesondere mittels (Neuro)Fuzzy-Logik oder einem neuronalen Netzwerk, auf einfache und besonders effektive Art und Weise der jeweilige Grad der Übereinstimmungen festgestellt und die entsprechenden Graphoelemente und/oder andere Kenngrößen differentialdiagnostisch geordnet werden. Die Referenzdatensätze werden zweckmäßigerweise durch den jeweils aktuellen Datensatz in der Art einer selbstlernenden Wissensbasis ergänzt. Dabei ermöglicht eine jedem Referenzdatensatz zugeordnete Kennung für ein entsprechendes Krankheitsbild oder -muster die Auswertung der entsprechenden Messung im Hinblick auf eine spätere Diagnose. Eine zudem anhand der jeweiligen Übereinstimmung des Datensatzes mit mehreren Referenzdatensätzen ermittelte Kennung ermöglicht darüber hinaus eine Wahrscheinlichkeitsaussage zu jedem von mehreren prädiagnostizierten Krankheitsbildern.

Bezüglich der Vorrichtung wird die Aufgabe erfindungsgemäß gelöst durch die Merkmale des Anspruchs 14. Vorteilhafte Ausgestaltungen sind in den auf diesen rückbezogenen Unteransprüchen angegeben.

Eine mit einer Empfängeranordnung zur Aufnahme der Ortskurve jedes Markierers verbundene Datenverarbeitungsanlage weist dazu eine Verarbeitugsstufe zur Berechnung eines die Ortskurve repräsentierenden Datensatzes aus Signalen der Empfängeranordnung auf. Diese kann eine Anzahl von im Raum verteilt zueinander angeordnete Ultraschallsende- und -empfangsgeräte, CCD-Kamaras (Video-Kamaras) Photoelemente oder drgl. zur Aufnahme und ggf. Vorverarbeitung von akustischen oder optischen Signalen sein. Die entsprechenden Empfänger sind zweckmäßigerweise zueinander rechtwinklig angeordnet, so daß eine Aufnahhme der Ortskurven der Markierer in zumindest zwei unterschiedlichen Ebenen, z. B. der xy-Ebene und der yz-Ebene oder xz-Ebene, erfolgt. Die Koordinatendaten der Ortskurve bezüglich der dritten Ebene können dann aus den Meßdaten der beiden Empfänger berechnet werden. Bei Verwendung von Ultraschall anstelle von Licht zur Markierung kann die Messung auch in einem nicht abgedunkelten Raum ausgeführt werden.

Eine der Verarbeitungsstufe nachgeordnete Datenbank dient zur Hinterlegung von Referenzdatensätzen, die vorzugsweise in einer Vielzahl von Referenzmessungen ermittelt worden sind. Ein Analysemodul oder eine Analysestufe der Datenverarbeitungsanlage ermittelt aus dem aktuell erfaßten Datensatz und aus dem entsprechenden Referenzdatensatz eine Anzahl von Kenngrößen bzw. Referenzgrößen, die in einem Vergleichsmodul oder in einer Vergleichsstufe anhand der Kenn- und Referenzgrößen in der Art einer Mustererkennung den Grad der Übereinstimmung zwischen den Datensätzen ermittelt. Die Datenverarbeitungsanlage ordnet anschließend jedem Datensatz eine einem Krankheitsbild entsprechende Kennung zu und übergibt den Datensatz anhand der Kennung der Datenbank zur Erweiterung des entsprechenden Referenzdatensatzes.

Die Verarbeitungsstufe ordnet die Ortskurve jedes Markierers zweckmäßigerweise als Datenfeld dem Datensatz zu. Dadurch ist eine Matrix mit einer der Anzahl der Markierer entsprechenden Anzahl von Datenfeldern gegeben, wobei jedes Datenfeld die drei Ortskoordinaten bezogen auf ein karthesisches Kooordinatensystem zum jeweiligen Zeitpunkt enthält. Der Verarbeitungsstufe ist vorteilhafterweise ein temporärer Datensatzspeicher zur Zwischenspeicherung der erfaßten Meßdaten nachgeordnet.

Die mit der Erfindung erzielten Vorteile bestehen insbesondere darin, daß durch eine rechnergestüzte Auswertung der bei einer Vielzahl von optisch oder akustisch erfaßten Bewegungsmustern ermittelten Meßdaten und die Interpretation daraus abgeleiteter Kurvenverläufe und Funktionsmuster anhand entsprechender Kenngrößen eine für eine Diagnose heranziehbare Aussage über das Krankheitsbild einer dem jeweiligen Bewegungsmuster zugrundeliegenden Störung getroffen werden kann. Während die Meßdatenerfassung praktisch berührungsfrei am Körper des Probanden erfolgt, erfolgt die Auswertung in einer vom Körper des Probanden losgelösten Datenverarbeitungsanlage, in der außerhalb des Körpers die Meßdaten verarbeitet und für eine Mustererkennung aufbereitet werden.

Die Auswertung von insbesondere Kopf-Körper-Bewegungsmustern sowohl unauffälliger Probanden als auch auffälliger Probanden typischer Krankheitsbilder ermöglicht das Anlegen einer Wissensbasis mit einer Vielzahl von Referenzdaten und -mustern, anhand derer aktuell erfaßte und nicht diagnostizierte Bewegungsmuster bekannten Krankheitsbildern zugeordnet werden können. Dadurch können qualitative und quantitative Aussagen insbesondere über psychische, psychosomatische und/oder neurologische Störungen, wie z. B. Schizophrenie, Depression und Parkinson-Syndrom, anhand der Auswertung der ermittelten Kenngrößen gemacht werden.

Ein Ausführungsbeispiel der Erfindung wird nachfolgend anhand einer Zeichnung näher beschrieben. Darin zeigen:
- Fig. 1: schematisch eine Vorrichtung mit zur Auswertung eines Bewegungsmusters vorgesehen Komponenten,
- Fig. 2: ein bei einer Schrittfolge eines Probanden erzeugtes Bewegungsmuster, repräsentiert durch Ortskurven von vier Markierern in einer Projektion auf die xy-Ebene
- Fig. 3: ein beim Stehen des Probanden erzeugtes Bewegungsmuster in einer Darstellung gemäß Fig. 2, und
- Fig.4: das sich infolge einer Therapie verändernde Bewegungsmuster eines diagnostizierten Krankheitsbildes.

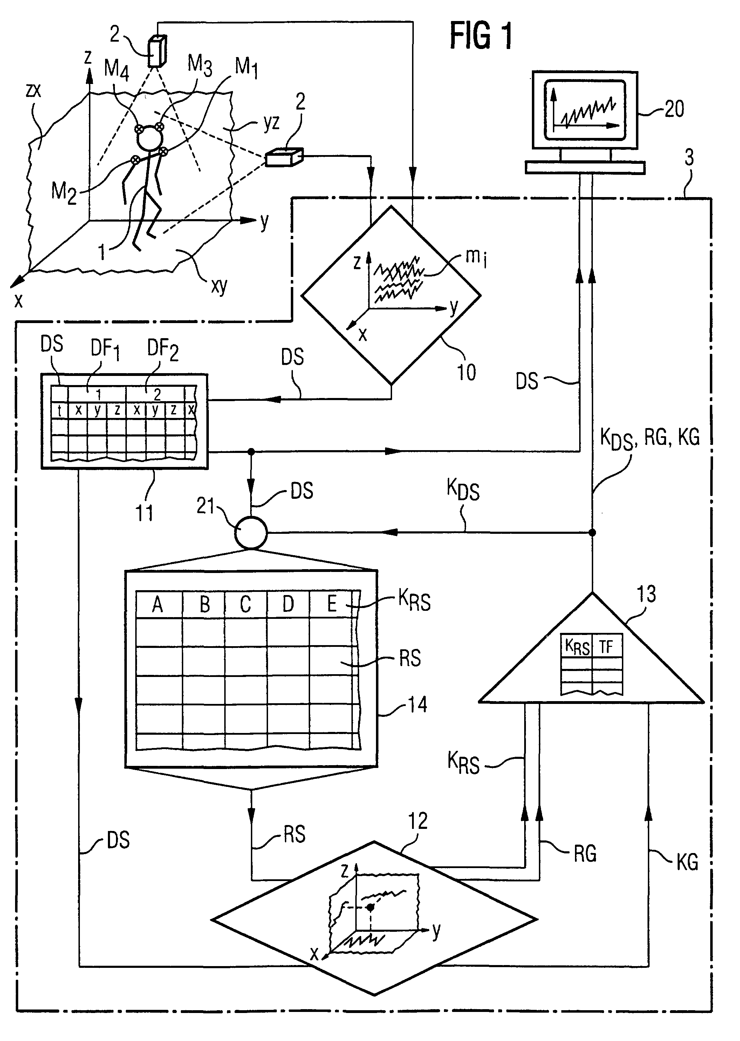

Zur Erfassung des Bewegungsmusters eines Probanden 1 sind gemäß Fig.1 zwei zueinander rechtwinklig ausgerichtete Empfänger 2 vorgesehen. Diese empfangen in nicht näher dargestellter Art und Weise Signale von einer Anzahl von mit dem Probanden 1 mitbewegten Markierern Mᵢ. Die Visualisierung der Körperbewegung kann besonders einfach auf optischem Wege realisiert werden. In diesem Fall verwendet man Glühlampen oder Leuchtdioden als Markierer Mᵢ und entsprechend je eine Kamera, wie z.B. eine Videokamera, als Empfänger 2. Eine Markierung des Bewegungsmusters ist auch mittels Ultraschallsendern als Markierer Mᵢ und Ultraschallempfängern als Empfänger 2 möglich. Alternativ können auch passive Markierer Mᵢ eingesetzt werden, die das von einer externen Quelle emittierte Signal lediglich reflektieren. Wie in der sogenannten Cranio-Corpo-Graphie üblich, beschränkt sich dabei die Beobachtung zweckmäßigerweise auf die Kopf- und Rumpfbewegung des Probanden 1. Dazu wird je ein Markierer M₁ und M_{2,} auf der linken bzw. der rechten Schulter des Probanden 1 angebracht, sowie je ein weiterer Markierer M₃ und M₄ über dessen Stirn bzw. dessen Hinterhaupt.

Die Empfänger 2 führen je ein zweidimensionales Bild der Bewegung der Markierer Mᵢ einer in einer Datenverarbeitungsanlage 3 enthaltenen Verarbeitungsstufe 10 zu, die aus den von den Empfängern 2 übermittelten Bildern die Ortskurve mᵢ jedes Markierers Mᵢ im dreidimensionalen Raum in Abhängigkeit von der Zeit t ermitteln. Die Raumkoordinaten jeder Ortskurve mᵢ werden in einem karthesischen Koordinatensystem x,y,z dargestellt, wobei die Ausgangsstellung des Probanden 1 dem Null-Punkt zugeordnet ist und damit die x-Achse der Lateralachse entspricht. Die y-Achse verläuft dann horizontal in Laufrichtung des Probanden 1, während sich die z-Achse vertikal nach oben erstreckt. Die von den jeweiligen Achsen gebildeten Grundebenen des Koordinatensystem sind die xy-Ebene (horizontal), die yz-Ebene (longitudinal vertikal) und die zx-Ebene (lateral vertikal).

Die Berechnung der Ortskurve mᵢ jedes Markierers Mᵢ erfolgt mittels der Datenverarbeitungsanlage 3 anhand eines Algorithmus der Verarbeitungsstufe 10. Falls für die Empfänger 2 eine analoge Aufzeichnungstechnik verwendet wird, findet in der Verarbeitungsstufe 10 zunächst eine Umwandlung von analogen Daten in digitale Daten statt. Die Verarbeitungstufe 10 übergibt die Ortskurven mᵢ als Datensatz DS einem vorzugsweise temporären Datensatzspeicher 11. Der Datensatz DS ist dabei in Datenfelder DFᵢ gegliedert, wobei ein Datenfeld DFᵢ die Ortskurve mᵢ eines Markierers Mᵢ repräsentiert.

Der Datensatzspeicher 11 stellt den Datensatz DS einem in Form von Software realisierten Analysemodul 12 zur Verfügung. Das Analysemodul 12 stellt zunächst durch Datenauswahl aus dem Datensatz DS jeweils eine Projektion der Ortskurven mᵢ auf die Grundebenen xy,yz und zx her. Da die Ortskurven mᵢ typischerweise eine durch eine Körperschwankung hervorgerufene periodische Struktur aufweisen, führt ein Algorithmus des Analysemoduls 12 weiter eine Unterteilung der Ortskurven mᵢ in periodische Sequenzen durch. Eine derartige Sequenz, deren Anfang und Ende jeweils durch eine scharfe Richtungsänderung der Ortskurve mᵢ gekennzeichnet ist, entspricht dabei genau einem Zyklus der Körperschwankung. Aus den auf die Grundebenen xy, yz und zx projezierten und in Sequenzen unterteilten Ortskurven mᵢ leiten weitere Algorithmen des Analysemoduls 12 ferner eine Anzahl von Kenngrößen KG ab.

Kenngrößen KG werden einerseits durch geometrische und physikalische Vermessung der Ortskurven mᵢ abgeleitet. Aufgrund der periodischen Struktur jeder Ortskurve mᵢ sind relevante Kenngrößen KG insbesondere die Amplitude, die Periodendauer, die Frequenz einer Schwankung und die während einer Schwankungsperiode quer zur Schwankungsrichtung zurückgelegte Strecke (Schrittweite). Diese Kenngrößen KG lassen sich sowohl aus einer einzelnen Sequenz ermitteln (single-step-Analyse) als auch aus einer Anzahl von Sequenzen statistisch ableiten und in Form von Mittelwert und Standardabweichung angeben (whole-reaction-Analyse). Des weiteren werden Unregelmäßigkeiten in der Körperschwankung durch Angabe einer Amplitudenverteilung und einer mittels Spektralanalyse (Fourriertransformation) gewonnenen Frequenzverteilung quantifiziert. Ferner werden physikalische Kenngrößen KG aus der Schwerpunktsbewegung des Körpers, der Drehung des Körpers gegenüber dem Raum sowie der Drehung des Kopfes relativ zum Rumpf ermittelt. Dazu werden die Ortskurven mᵢ mehrerer Markierer Mᵢ miteinander kombiniert.

Zusätzlich zu der physikalischen und/oder geometrischen Charakterisierung ermittelt das Analysemodul 12 andererseits die Übereinstimmung der Linienform der Ortskurven mᵢ mit als Grapho-Element hinterlegten Vergleichsmustern. Auch ein Vergleich mit einem Grapho-Element kann auf eine einzelne Sequenz oder die gesamte Ortskurve mᵢ bezogen sein. Dabei werden Schwankungssequenzen anhand der Form ihrer Umkehrbereiche charakterisiert. Typische Grapho-Elemente weisen bogen-, schleifen- oder spitzenförmige Umkehrbereiche auf. Grapho-Elemente zur Beschreibung der gesamten Ortskurve mᵢ orientieren sich dagegen an der Umrißform der von einer Projektion einer Ortskurve mᵢ überstrichenen Fläche. Diese wird mit Grapho-Elementen in Form geometrischer Figuren (z.B. Dreieck, Quadrat, etc.) oder vergleichsweise komplizierten Mustern (z.B. Schmetterlingsform) verglichen. Das Analysemodul 12 übergibt die aus dem Datensatz DS abgeleiteten Kenngrößen KG an ein Vergleichsmodul 13.

Zur Bewertung des Datensatzes DS wird dem Analysemodul 12 aus einer Datenbank 14 ein Referenzdatensatz RS zur Verfügung gestellt. Der Referenzdatensatz RS weist eine dem Datensatz DS entsprechende Datenstruktur auf, ist jedoch zusätzlich mit einer einem Krankheitsbild entsprechenden Kennung K_{RS} versehen. Das Analysemodul 12 ermittelt aus dem Referenzdatensatz RS eine Anzahl von Referenzgrößen RG, welche hinsichtlich ihrer Ableitung den Kenngrößen KG des Datensatzes DS entsprechen. Die Referenzgrößen RG werden nach deren Ableitung ebenfalls dem Vergleichsmodul 13 zugeleitet. Die Erstellung des Referenzdatensatzes RS erfolgt in einer Referenzmessung analog zur Erstellung des Datzensatzes DS. Zusammen mit den Referenzgrößen RG wird dem Vergleichsmodul 13 die Kennung K_{RS} des zugehörigen Referenzdatensatzes RS übermittelt. Im Vergleichsmodul 13 wird anhand der Kenngrößen KG und der Referenzgrößen RG der Grad der Übereinstimmung zwischen dem Datensatz DS und dem Referenzdatensatz RS ermittelt. Indem bestimmte Grapho-Elemente typischen Krankeitsbildern zugeordnet sind, wird anhand eines direkten Mustervergleichs ein aktuelles Bewegungsmuster direkt typisiert oder zumindest qualitativ eingeordnet. Unter Heranziehung weiterer Kenngrößen KG wird die Zuordnung quantitativ gestützt.

Die pathologischen Eigenschaften des Bewegungsmusters eines Probanden 1 sind stets überlagert mit individuellen Eigenschaften. Des weiteren können Elemente oder Symptome - und damit einzelne Kenngrößen KG - der Bewegungsmuster unterschiedlicher Krankheitsbilder gleich oder ähnlich sein. Ferner ist eine an einem Probanden 1 durchgeführte Untersuchung nicht exakt reproduzierbar. Sowohl die Kenngrößen KG als auch die Referenzgrößen RG sind daher in der Regel hinsichtlich ihrer prädiagnostischen Aussagekraft unscharf. Um dieser Unschärfe Rechnung zu tragen, arbeitet das Vergleichsmodul 13 vorzugsweise auf der Basis der sogenannten Fuzzy-Logik. Gemäß den Regeln der Fuzzy-Logik wird eine als exakter Meßwert eingegebene Kenngröße KG oder Referenzgröße RG künstlich unscharf gemacht (fuzzifiziert). Durch Vergleich einer unscharfen Kenngröße KG mit einer unscharfen Referenzgröße RG wird im Vergleichsmodul 13 ein den Grad der Übereinstimmung angebender Typisierungsfaktor TF ermittelt. Der Typisierungsfaktor TF spiegelt infolge der Fuzzyfizierung verstärkt die pathologische Übereinstimmung des Datensatzes DS mit dem Referenzdatensatz RS wieder. Individuelle und irreproduzierbare Details im Bewegungsmuster werden durch die unscharfe Betrachtungsweise mittels der Fuzzy-Logik herausgefiltert. Dies spiegelt sich in der Angabe einer Wahrscheinlichkeit der Übereinstimmung mit dem Referenzdatensatz RS bei einer anschließenden Defuzzifizierung wieder.

Zur Ermittlung einer einem Krankheitsbild entsprechenden Kennung K_{DS} für den Datensatz DS führt das Vergleichsmodul 13 auf die beschriebene Weise einen Vergleich des Datensatzes DS mit einer Vielzahl von der Datenbank 14 entnommenen Referenzdatensätzen RS durch. Die bei jedem Einzelvergleich eines Datensatzes DS mit einem Referenzdatensatz RS gewonnenen Typisierungsfaktoren TF werden abschließend überlagert und zur Ermittlung der Kennung K_{DS} herangezogen. Zur Ermittlung der Kennung K_{DS} wird dabei z. B. ein neuronales Netzwerk verwendet.

Das Vergleichsmodul 13 ist zur Ausgabe der Kennung K_{DS} mit einem Ausgabemodul 20, z. B. einem Bildschirm, einem Drucker oder einem Plotter, verbunden. Über dieses Ausgabemodul 20 kann weiterhin das im Datensatzspeicher 11 in Form des Datensatzes DS niedergelegte Bewegungsmuster ausgegeben werden. Abschließend wird der Datensatz mittels einer Datenbankeingabesteuerung 21 mit der zugehörigen Kennung K_{DS} versehen und entsprechend dieser Kennung K_{DS} nach Art einer selbstlernenden Wissensbasis der Datenbank 14 als Referenzdatensatz RS hinzugefügt.

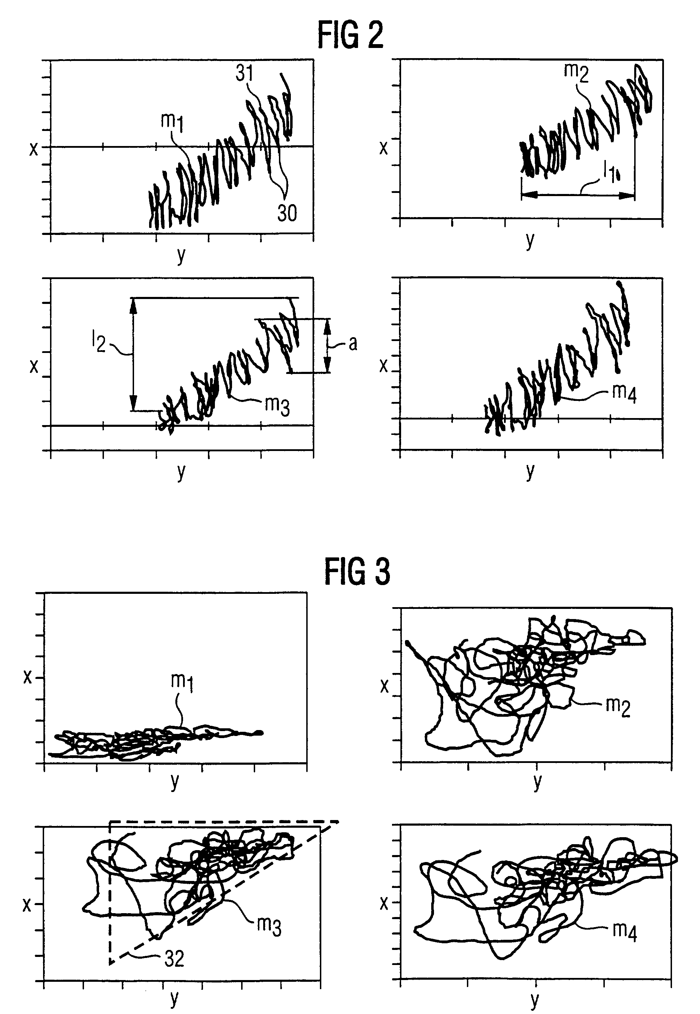

Fig. 2 zeigt ein für einen Schritt-Test nach Unterberger/Fukuda typisches Bewegungsmuster anhand der Ortskurven m₁ bis m₄ der Markierer M₁ bis M₄ in einer Projektion auf die horizontale Ebene xy. Bei einem derartigen Schritt-Test führt der Proband 1 eine Schrittbewegung auf der Stelle aus. Üblicherweise werden dem Probanden 1 dazu die Augen verbunden, um eine visuelle Orientierung im Raum zu verhindern. Die Ortskurven mᵢ zeigen eine ausgeprägte wellenartige Periodizität, die durch die Gewichtsverlagerung des Körpers beim Schreiten hervorgerufen wird. Eine durch zwei benachbarte Umkehrpunkte 30 eingegrenzte Sequenz 31, wie sie in Fig. 2 beispielhaft an der Ortskurve m₁ dargestellt ist, repräsentiert somit einen Schrittzyklus des Probanden 1 mit zwei aufeinanderfolgenden Schritten.

Typischer Weise propagiert auch ein unauffälliger Proband 1 beim Schritt-Test in Richtung der longitudinalen Achse y um eine Strecke I₁. Eine laterale Abweichung I₂ des Körperschwerpunkts oder die Amplitude a der Lateralschwankung werden bei Übersschreiten eines (kritischen) Schwellwertes als pathologisch eingestuft. Ein Vergleich der Ortskurven mᵢ gemäß Fig. 2 mit den genannten sequentiellen Grapho-Elementen ergibt eine hohe Übereinstimmung mit einem spitzenförmigen Vergleichsmuster.

In Fig. 3 ist ein Bewegungsmuster desselben Probanden 1 gezeigt. Jedoch wurde dieses Bewegungsmuster in einem Steh-Test nach Romberg erzeugt. Hierbei steht der Proband 1 etwa 1 Minute lang, wobei üblicherweise ebenfalls die Augen verbunden sind. Das bei einem Steh-Test erzeugte Bewegungsmuster gleicht einer chaotisch verzerrten Kreisbewegung mit im Vergleich zum Schritt-Test kleiner Amplitude (der in Fig. 3 verwendete Abbildungsmaßstab ist gegenüber dem Abbildungsmaßstab gemäß Fig. 2 etwa 10-fach vergrößert). Die bei einem Steh-Test erzeugte Ortskurve mᵢ gemäß Fig. 3 wird bevorzugt in ihrer Gesamtheit anhand von Grapho-Elementen charakterisiert. Für diese Ortskurven mᵢ ergibt sich ihrer Umrißform gemäß eine hohe Übereinstimmung mit dem ebenfalls in Fig. 3 beispielhaft eingezeichneten rechtwinkligen Dreieck 32.

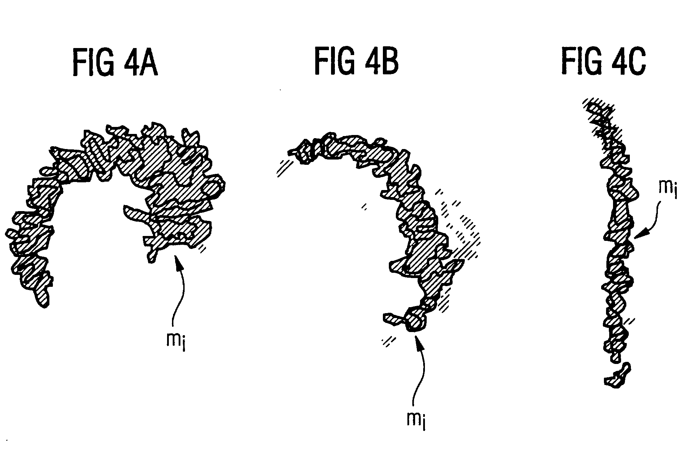

Zusätzlich zur Erkennung eines Krankheitsbildes kann das Verfahren auch als therapiebegleitende Untersuchungsmethode eingesetzt werden. Fig. 4 zeigt die Aufnahme eines mehrfach an einem Schizophreniepatienten durchgeführten Schritt-Test, wobei das in den Figuren 4a, 4b und 4c gezeigte Bewegungsmuster jeweils im Abstand von 30 Tagen aufgenommen wurden. Der während der Therapie erzielte Behandlungserfolg zeigt sich in der von Fig. 4a sukzessiv nach Fig. 4c abnehmenden Linkskrümmung der Ortskurven mᵢ. Das Verfahren wird daher besonders vorteilhaft als Meßmethode zur Überprüfung der medikamentösen Einstellung eines Patienten hinzugezogen.

### Bezugszeichenliste

- 1: Proband
- 2: Empfänger
- 3: Datenverarbeitungsanlage
- 10: Verarbeitungsstufe
- 11: Datensatzspeicher
- 12: Analysemodul
- 13: Vergleichsmodul
- 20: Ausgabemodul
- 21: Datenbankeingabesteuerung
- 30: Umkehrpunkt
- 31: Sequenz
- 32: Dreieck als Grapho-Element

- Mᵢ: Ortskurve
- DS: Datensatz
- DFᵢ: Datenfeld
- KG: Kenngröße
- RS: Referenzdatensatz
- K_{RS}: Kennung des Referenzdatensatzes
- RG: Referenzgröße
- TF: Typisierungsfaktor
- K_{DS}: Kennung des Datensatzes
- x: laterale Achse
- y: longitudinale Achse
- z: vertikale Achse
- xy: horizontale Ebene
- yz: longitudinal-vertikale Ebene
- zx: lateral-vertikale Ebene
- I₁: longitudinale Schwerpunktsbewegung
- I₂: Lateralabweichung des Schwerpunkts
- a: Amplitude der Lateralschwankung

## Patentansprüche

1. Verfahren zur Auswertung eines Kopf-Rumpf-Bewegungsmusters, das anhand einer Anzahl von mit dem im Raum beweglichen Körper eines Probanden (1) mitbewegten Markierern (Mᵢ) aufgenommen wird,
- wobei die Ortskurve (mᵢ) jedes Markierers (Mᵢ) im dreidimensionalen Raum in Abhängigkeit von der Zeit (t) erfaßt und als Datenfeld (DFᵢ) eines allen Markierern (Mᵢ) gemeinsamen Datensatzes (DS) gespeichert wird,
- wobei das Bewegungsmuster anhand von aus dem Datensatz (DS) abgeleiteten Kenngrößen (KG) charakterisiert wird,
- wobei jede Kenngröße (KG) mit einer aus einem hinterlegten Referenzdatensatz (RS) entsprechend abgeleiteten Referenzgröße (RG) verglichen wird, und
- wobei die oder jede Kenngröße (KG) aus einer Projektion mindestens einer Ortskurve (mᵢ) auf eine der drei Grundebenen (xy, yz, zx) eines karthesischen Koordinatensystems abgeleitet wird.

2. Verfahren nach Anspruch 1, bei dem aus mindestens einer Ortskurve (mᵢ) jeweils einem Schwankungszyklus der Körperschwankung entsprechende Sequenzen ermittelt werden, wobei aus einer einzelnen Sequenz mindestens eine Kenngröße (KG) abgeleitet wird.

3. Verfahren nach Anspruch 2, bei dem der Mittelwert und die Standardabweichung der Kenngröße (KG) aus einer Anzahl von Sequenzen abgeleitet wird.

4. Verfahren nach einem der Ansprüche 1 bis 3, bei dem aus der Periodizität mindestens einer Ortskurve (mᵢ) die Amplitudenverteilung und/oder die Frequenzverteilung einer Körperschwankung als Kenngröße (KG) ermittelt wird.

5. Verfahren nach einem der Ansprüche 1 bis 4, bei dem aus dem Abstand (I₁,I₂) zwischen einer Anfangsposition und einer Endposition mindestens eines Markierers (Mᵢ) die Schwerpunktsbewegung des Körpers als Kenngröße (KG) ermittelt wird.

6. Verfahren nach einem der Ansprüche 1 bis 5, bei dem anhand der Positionen der Markierer (Mᵢ) die Ausrichtung mindestens eines Körperteils im Raum als Kenngröße (KG) ermittelt wird.

7. Verfahren nach einem der Ansprüche 1 bis 6, bei dem anhand der Positionen der Markierer (Mᵢ) die Stellung zwischen Teilen des Körpers zueinander als Kenngröße (KG) ermittelt wird.

8. Verfahren nach einem der Ansprüche 1 bis 7, bei dem die Länge mindestens einer Ortskurve (mᵢ) als Kenngröße (KG) ermittelt wird.

9. Verfahren nach einem der Ansprüche 1 bis 8, bei dem der Grad der Übereinstimmung zwischen einem als Grapho-Element hinterlegten Muster und der Form mindestens eines Teils der Ortskurve (mᵢ) als Kenngröße (KG) ermittelt wird.

10. Verfahren nach einem der Ansprüche 1 bis 9, bei dem ein den Grad der Übereinstimmung zwischen dem Datensatz (DS) und dem Referenzdatensatz (RS) angebender Typisierungsfaktor (TF) mittels Fuzzy-Logik ermittelt wird.

11. Verfahren nach einem derAnsprüche 1 bis 10, bei dem jedem in einer Datenbank (14) hinterlegten Referenzdatensatz (RS) eine einem Krankheitsbild entsprechende Kennung (K_{RS}) zugeordnet ist, wobei anhand der jeweiligen Übereinstimmung des Datensatzes (DS) mit mehreren Referenzdatensätzen (RS) für den Datensatz (DS) eine Kennung (K_{DS}) ermittelt wird.

12. Verfahren nach Anspruch 11, bei dem die Kennung (K_{DS}) mittels eines neuronalen Netzes ermittelt wird.

13. Verfahren nach Anspruch 11 oder 12, bei dem nach Art einer selbstlernenden Wissensbasis jeder Datensatz (DS) anhand dessen Kennung (K_{DS}) der Datenbank (14) hinzugefügt wird.

14. Vorrichtung zur Durchführung des Verfahrens nach einem der Ansprüche 1 bis 13, mit einer Anzahl von am Kopf und am Körper eines Probanden anbringbaren Markierern (Mᵢ) und mit einer zwei zueinander rechtwinklig angeordnete Empfänger (2) umfassenden Empfängeranordnung (2) zur Aufnahme der Ortskurve (mᵢ) jedes Markierers (Mᵢ) sowie mit einer mit der Empfangsanordnung (2) verbundenen Datenverarbeitungsanlage (3), die
- eine Verarbeitungsstufe (10) zur Berechnung eines die Ortskurve (m;) repräsentierenden Datensatz (DS) aus Signalen der Empfängeranordnung (2),
- eine eine Anzahl von Referenzdatensätzen (RS) enthaltende Datenbank (14),
- ein Analysemodul (12) zur Erstellung von Kenngrößen (KG) aus dem Datensatz (DS) und von Referenzgrößen (RG) aus dem Referenzdatensatz (RS), und
- ein Vergleichsmodul (13) umfaßt, das anhand der Kenngrößen (KG) und der Referenzgrößen (RG) den Grad der Übereinstimmung zwischen dem Datensatz (DS) und dem Referenzdatensatz (RS) ermittelt.

15. Vorrichtung nach Anspruch 14,
**dadurch gekennzeichnet,**
**daß** die Verarbeitungsstufe (10) die Ortskurve (mᵢ) jedes Markierers (Mᵢ) als Datenfeld (DFᵢ) dem Datensatz (DS) zuordnet.

16. Vorrichtung nach Anspruch 14 oder 15,
**dadurch gekennzeichnet,**
**daß** der Verarbeitungsstufe (10) ein temporärer Datensatzspeicher (11) nachgeordnet ist.

17. Vorrichtung nach einem der Ansprüche 14 bis 16,
**dadurch gekennzeichnet,**
**daß** das Vergleichsmodul (13) jedem Datensatz (DS) eine einem Krankheitsbild entsprechende Kennung (K_{DS}) zuordnet und den Datensatz (DS) anhand der Kennung (K_{DS}) der Datenbank (14) zuführt.

18. Vorrichtung nach einem der Ansprüche 14 bis 17,
**dadurch gekennzeichnet,**
**daß** die Datenverarbeitungsanlage (3) die oder jede Kenngröße (KG) einem Ausgabemodul (20) zur Darstellung des Bewegungsmusters und/oder eines Referenzmusters zuführt.

## Claims

1. Method for evaluating a head/trunk movement pattern recorded using a number of markers (Mᵢ) moving together with the body (which can move in space) of a subject (1),
- the locus curve (mᵢ) for each marker (Mᵢ) being detected in three-dimensional space as a function of time (t) and being stored as a data field (DFᵢ) of a data record (DS) which is common to all markers (Mᵢ),
- the movement pattern being characterized using characteristic variables (KG) derived from the data record (DS),
- each characteristic variable (KG) being compared with a reference variable (RG) derived accordingly from a stored reference data record (RS), and
- the or each characteristic variable (KG) being derived from a projection of at least one locus curve (mᵢ) onto one of the three datum planes (xy, yz, zx) of a Cartesian coordinate system.

2. Method according to Claim 1, in which at least one locus curve (mᵢ) is used to ascertain sequences each corresponding to a sway cycle of the body sway, at least one characteristic variable (KG) being derived from an individual sequence.

3. Method according to Claim 2, in which the mean and the standard deviation of the characteristic variable (KG) are derived from a number of sequences.

4. Method according to one of Claims 1 to 3, in which the amplitude distribution and/or the frequency distribution of any body sway is ascertained as a characteristic variable (KG) from the periodicity of at least one locus curve (mᵢ).

5. Method according to one of Claims 1 to 4, in which the movement of the body's centre of gravity is ascertained as a characteristic variable (KG) from the distance (I₁, I₂) between a starting position and a finishing position of at least one marker (Mᵢ).

6. Method according to one of Claims 1 to 5, in which the positions of the markers (Mᵢ) are used to ascertain the orientation of at least one body part in space as a characteristic variable (KG).

7. Method according to one of Claims 1 to 6, in which the positions of the markers (Mᵢ) are used to ascertain the attitude of parts of the body with respect to one another as a characteristic variable (KG).

8. Method according to one of Claims 1 to 7, in which the length of at least one locus curve (mᵢ) is ascertained as a characteristic variable (KG).

9. Method according to one of Claims 1 to 8, in which the degree of correspondence between a pattern stored as a graphical element and the shape of at least one part of the locus curve (mᵢ) is ascertained as a characteristic variable (KG).

10. Method according to one of Claims 1 to 9, in which a standardization factor (TF) indicating the degree of correspondence between the data record (DS) and the reference data record (RS) is ascertained using fuzzy logic.

11. Method according to one of Claims 1 to 10, in which each reference data record (RS) stored in a database (14) has an associated identifier (K_{RS}) based on a clinical picture, the respective correspondence of the data record (DS) to a plurality of reference data records (RS) being used to ascertain an identifier (K_{DS}) for the data record (DS).

12. Method according to Claim 11, in which the identifier (K_{DS}) is ascertained using a neural network.

13. Method according to Claim 11 or 12, in which the identifier (K_{DS}) for each data record (DS) is used to add said data record (DS) to the database (14) in the style of a self-learning knowledge base.

14. Apparatus for carrying out the method according to one of Claims 1 to 13, having a number of markers (Mᵢ) which can be attached to the head and to the body of a subject, and having a receiver arrangement (2), comprising two receivers (2) arranged at right angles to one another, for recording the locus curve (mᵢ) for each marker (Mᵢ) and having a data processing system (3) which is connected to the reception arrangement (2) and which comprises
- a processing stage (10) for calculating a data record (DS) representing the locus curve (mᵢ) from signals from the receiver arrangement (2),
- a database (14) containing a number of reference data records (RS),
- an analysis module (12) for creating characteristic variables (KG) from the data record (DS) and for creating reference variables (RG) from the reference data record (RS), and
- a comparison module (13) which uses the characteristic variables (KG) and the reference variables (RG) to ascertain the degree of correspondence between the data record (DS) and the reference data record (RS).

15. Apparatus according to Claim 14, **characterized in that** the processing stage (10) associates the locus curve (mᵢ) for each marker (Mᵢ) with the data record (DS) as a data field (DFᵢ).

16. Apparatus according to Claim 14 or 15, **characterized in that** the processing stage (10) has a temporary data record store (11) arranged downstream of it.

17. Apparatus according to one of Claims 14 to 16, **characterized in that** the comparison module (13) associates with each data record (DS) an identifier (K_{DS}) based on a clinical picture and supplies the data record (DS) to the database (14) using the identifier (K_{DS}).

18. Apparatus according to one of Claims 14 to 17, **characterized in that** the data processing system (3) supplies the or each characteristic variable (KG) to an output module (20) for displaying the movement pattern and/or a reference pattern.

## Revendications

1. Procédé pour évaluer modèle de déplacement de la tête et du tronc, qui est enregistré sur la base d'un certain nombre d'éléments de marquage (Mᵢ) déplacés conjointement avec le corps, mobile dans l'espace, d'un sujet de test (1),
- selon lequel la courbe de lieu (mᵢ) de chaque élément de marquage (Mᵢ) est détectée dans l'espace tridimensionnel en fonction du temps (t) et est mémorisée en tant que champ de données (DFᵢ) d'un ensemble de données (DS) commun à tous les éléments de marquage (Mᵢ),
- selon lequel le modèle de déplacement est caractérisé sur la base de grandeurs caractéristiques (KG) dérivées de l'ensemble de données (DS),
- selon lequel chaque grandeur caractéristiques (KG) est comparée à une grandeur de référence (RG) dérivée de façon correspondante d'un ensemble mémorisé de données de référence (RS), et
- selon lequel la ou chaque grandeur caractéristique (KG) est dérivée d'une projection d'au moins une courbe de lieu (mᵢ) dans l'un des trois plan de base (xy, yz, zx) d'un système de coordonnées cartésien.

2. Procédé selon la revendication 1, selon lequel les séquences correspondant chacune à un cycle de balancement du corps sont déterminées à partir d'au moins une courbe de lieu (mᵢ), au moins une grandeur caractéristique (KG) étant dérivée d'une séquence individuelle.

3. Procédé selon la revendication 2, selon lequel la valeur moyenne et l'écart-type de la grandeur caractéristique (KG) sont dérivés d'un certain nombre de séquences.

4. Procédé selon l'une des revendications 1 à 3, selon lequel la distribution d'amplitude et/ou la distribution de fréquences d'une oscillation du corps est déterminée en tant que grandeur caractéristique (KG) à partir de la périodicité d'au moins une courbe de lieu (mᵢ).

5. Procédé selon l'une des revendications 1 à 4, selon lequel le déplacement du centre de gravité du corps est déterminé en tant que grandeur caractéristique (KG) à partir de la distance (I₁, I₂) entre une position de départe et une position finale d'au moins un élément de marquage (Mᵢ).

6. Procédé selon l'une des revendications 1 à 5, selon lequel l'orientation d'au moins une partie du corps dans l'espace est déterminée en tant que grandeur caractéristique (KG) à partir des positions des éléments de marquage (Mᵢ).

7. Procédé selon l'une des revendications 1 à 6, selon lequel la position entre des parties du corps est déterminée en tant que grandeur caractéristique à partir des positions des éléments de marquage (Mᵢ).

8. Procédé selon l'une des revendications 1 à 7, selon lequel la longueur d'au moins une courbe de lieu (mᵢ) est déterminée en tant que grandeur caractéristique (KG).

9. Procédé selon l'une des revendications 1 à 8, selon lequel le degré de concordance entre un modèle mémorisé en tant qu'élément graphique et la forme d'au moins une partie de la courbe de lieu (mᵢ) est déterminé en tant que grandeur caractéristique (KG).

10. Procédé selon l'une des revendications 1 à 9, selon lequel un facteur de détermination de type (TF), qui indique la concordance entre l'ensemble de données (DS) et l'ensemble de données de référence (RS), est déterminé au moyen d'une logique floue.

11. Procédé selon l'une des revendications 1 à 10, selon lequel chaque ensemble de données de référence (RS) mémorisé dans une banque de données (14) est associée une caractéristique (K_{RS}) qui correspond à une image de maladie, auquel cas une caractérisation (K_{DS}) est déterminée sur la base de la concordance respective de l'ensemble de données (DS), avec plusieurs ensembles de données de référence (RS) et pour l'ensemble de données (DS).

12. Procédé selon la revendication 11, selon lequel la caractérisation (K_{DS}) est déterminée à l'aide d'un réseau neuronal.

13. Procédé selon la revendication 11 ou 12, selon lequel chaque ensemble de données (DS) est ajouté, sur la base de sa caractéristique (K_{DS}), à la banque de données (14), à la manière d'une base de connaissance à auto-apprentissage.

14. Dispositif pour la mise en oeuvre du procédé selon l'une des revendications 1 à 13, comportant un certain nombre d'éléments de marquage (Mᵢ) pouvant être appliqués sur la tête et sur le corps d'un sujet de test et comportant un dispositif de réception (2) qui comprend deux récepteurs (2) disposés perpendiculairement l'un à l'autre, et servant à enregistrer la courbe de lieu (mᵢ) de chaque élément de marquage (Mᵢ), ainsi qu'une installation de traitement de données (30) reliée au dispositif de réception (2) et qui comprend
- un étage de traitement (12) pour calculer un ensemble de données (DS) représentant la courbe de lieu (mᵢ) à partir de signaux du dispositif de réception (2),
- une banque de données (14) contenant un certain nombre d'ensembles de données de référence (RS),
- un module d'analyse (10) pour régler des grandeurs caractéristiques (KG) à partir de l'ensemble de données (DS) et de grandeurs de référence (RG) à partir de l'ensemble de données de référence (RF), et
- un module de comparaison (13), qui détermine, sur la base des grandeurs caractéristiques (KG) et des grandeurs de référence (RG), le degré de concordance entre l'ensemble de données (DS) et l'ensemble de données de référence (RS).

15. Dispositif selon la revendication 14, **caractérisé en ce que** l'étage de traitement (10) associe la courbe de lieu (mᵢ) de chaque élément de marquage (Mᵢ) en tant que champ de données (DSᵢ) à l'ensemble de données (DS).

16. Dispositif selon la revendication 14 ou 15, **caractérisé en ce qu'**une mémoire temporaire (11) d'ensembles de données est disposée en aval de l'étage de traitement (10).

17. Dispositif selon l'une des revendications 14 à 16, **caractérisé en ce que** le module de comparaison (13) associe à chaque ensemble de données (DS) une caractérisation (K_{DS}) qui correspond à une image de maladie et ensemble l'ensemble de données (DS) à la banque de données (14) sur la base de la caractérisation (K_{DS}).

18. Dispositif selon l'une des revendications 14 à 17, **caractérisé en ce que** l'installation de traitement de données (3) envoie la ou les grandeurs caractéristiques (KG) à un module de sortie (20) pour la représentation du modèle de déplacement et/ou d'un modèle de référence.
